# EUROPEAN PATENT SPECIFICATION

(11) **EP 4 165 663 B1**
(45) Date of publication and mention of the grant of the patent: **27.11.2024**
(21) Application number: 21732043.1
(22) Date of filing: 11.06.2021
(51) Int. Cl.: G21F 3/00, A61B 6/10

(54) **RADIATION SHIELD DEVICE**
STRAHLUNGSABSCHIRMUNGSVORRICHTUNG
DISPOSITIF DE PROTECTION CONTRE LES RAYONNEMENTS

(30) Priority: 12.06.2020 GB 202008931
(43) Date of publication of application: 19.04.2023
(73) Proprietor: Vestlandets Innovasjons selskap AS (VIS), 5006 Bergen (NO)
(72) Inventor: TUSETH, Vegard, 5019 Bergen (NO); DAVIDSEN, Cedric, 4602 Cheratte (FR)
(74) Representative: Hoffmann Eitle
(86) International application number: PCT/EP2021/065834
(87) International publication number: WO 2021/250255

(56) References cited:
- EP-A1- 2 904 614
- EP-A2- 3 185 246
- WO-A2-2005/104942
- DE-A1- 19 619 297
- US-A1- 2019 374 793
- US-B2- 7 057 194

## Description

The present invention relates to the protection against exposure to ionising radiation, more particularly to protective equipment in the field of radiation- and radiation-guided therapy, diagnosis and prognosis. Ionising radiations, such as x-rays and gamma rays, are commonly used to diagnose, prognose and treat medical conditions. Examples of treatments using ionising radiation include radiation therapy for cancer patients, whereas fluoroscopy, x-ray imaging, CT scanning and nuclear imaging are routinely used in hospitals, medical and dental clinics, and in medical research to visualise the body's internal organs and structures.

While radiation therapy and imaging are a crucial asset to modern medicine, these high-energy, highly penetrating radiation damage living cells. At levels experienced during an imaging session, the damage can be repaired by the cells. However, in cases of repeated or high-level exposure, the cells are no longer able to repair the damage, so that some cells will mutate into cancer cells or dysfunctional non-cancerous cells, or die. Overexposure to radiation may contribute and even lead to serious conditions, including cancers and acute radiation syndrome (ARS), and directives, regulations and protocols are in place to safeguard the health and safety of patients and health professionals.

The three fundamental principles for ionising radiation safety are time, distance, and shielding. With regard to the latter, Personal Protective Equipment (PPE) plays an important role in the protection of health professionals against radiation exposure. Radiation protective clothing conventionally comprises heavy metals, usually lead, but because of the toxicity of lead and environmental concerns, these are being progressively replaced by lead-free materials and composites (with predetermined lead-equivalence values). Such protective clothing includes aprons, gloves, head and eye wear. Lead aprons are one of the key parts of personal radiation protection equipment along with lead gloves, lead glasses, and thyroid shields or collars.

Typically, these PPE do not cover the whole body surface of the wearer, who consequently is exposed to direct and scattered radiation. This is particularly problematic in procedures where the health professionals must remain in close proximity to the radiation source. In these situations, lead protective clothing do not provide sufficient shielding against harmful radiation.

Another disadvantage of lead PPE is its bulkiness and weight which hinder the movements of the wearer, and increase the risk of orthopaedic problems when worn for lengths of time. The discomfort of the professional is further accentuated by lack of ventilation and breathability of the PPE. Consequently, there is a need for an alternative to wearable PPE to protection medical staff from harmful radiation.

Patients, on the other hand, must be exposed to the radiation to enable efficient imaging or therapy. However, the procedures are generally localised (e.g. localised surgery, localised tumour), and hence so should the exposure be. Protective equipment, such as goggles, and lead blankets or shields (such as breast shields) are available to protect sensitive and/or areas which are not to be irradiated, and blankets may be used to limit the radiation from reaching the health professional. However, blankets do not allow easy access, or any access at all, to covered parts of the patient's body, and are not suitable for procedures involving multiple target areas.

It is an object of this invention to mitigate problems such as those described above and to provide an improved alternative to existing products.

According to a first aspect of the invention, there is provided a radiation shield device according to claim 1.

The present invention is particularly advantageous in that it prevents radiation emanating from the radiation source from reaching the health professionals, and provides additional protection to the personal present in the treatment area. The shield device covers the patient but allows selective access to the target areas of the patient, whilst still shielding the health professional from radiation.

The flaps are preferably arranged and configured to be moved individually from a "shielding configuration" (or "shield configuration") in which it is in the plane of the sheet and covering the patient, to a "working configuration" in which it is folded over the sheet to expose the target area.

Preferably, the sheet comprises one or more pre-formed lines about which the flaps can be folded. The pre-formed lines enable the user to easily flip the flap open to uncover the target area, and to maintain the flap in the working configuration, in which the target area is exposed. The pre-formed lines may be located in or on a flap, in or on the sheet, or between a flap and the sheet, its primary (but not only) functions being to provide a guiding line for folding, and to stabilise the device over the patient.

In one embodiment, the rigidity of the sheet material is greater than the rigidity of the pre-formed lines. In this embodiment, the pre-formed lines are more flexible than the material of the sheet, thereby forming a line of weakness about which the flap can be folded onto the sheet. A line of weakness is preferably an area of the sheet, wherein the thickness is smaller than the thickness of the remainder of the sheet.

Alternatively, one or more of the pre-formed lines or folding lines may be devoid of sheet material. For example, the pre-formed lines or folding lines may comprise or consist of one or more spines or battens as described in further detail hereinbelow. This configuration maintains the integrity of the sheet material and reduce or prevent tear and breakage.

In another embodiment, the rigidity of the pre-formed lines is greater than the rigidity of the sheet material. In this embodiment, the pre-formed lines are more rigid than the material of the sheet, thereby acting as a spine about which the flap can be folded onto the sheet.

The device according to the present invention may comprise one or more relatively rigid folding lines and/or one or more relatively flexible folding lines.

Preferably, the one or more pre-formed lines comprise a spine. The spine preferably comprises or consists of an elongated member about which the sheet and/or flap can be folded. The spine preferably comprises a member which is more rigid than the sheet material. Preferably, the pre-formed line comprises a pocket configured and arranged so as to accommodate a spine. The pocket is preferably formed in or on the sheet material. It may be integrally formed in or on the sheet or secured to the sheet.

The spine may be made of a rigid material. However, preferably, the spine is made of a bendable material. More preferably, the spine is made of a material which can be bent to confer a desired shape to said spine. For example, the spine may be made of a flexible, bendable or shapable material, or a flexible, bendable or shapable structure, which enables the user to shape the spine, and therefore the sheet, as required during the procedure. The spine may comprise one section, or a plurality of sections. The latter configuration enables the flap to be folded completely or partially upon itself on upon the sheet.

The spine preferably comprises an elongated member. The spine may have a circular, rectangular, triangular or other cross-sectional shape. For example, the spine may be cylindrical, or made of a band of material.

Alternatively, the device does not comprise any pre-formed lines. Instead, the device comprises one or more spines or battens about which the device may be folded.

The weight of the shield may be adjusted depending on the intended use. A light sheet or blanket may be preferred for the comfort of the patient, but, if required, the weight of the shield may be increased to improve the stability of the shield on the patient. This may be effected by increasing the density of the sheet and/or of the spines. In other (non-medical) applications, the shield may be a mat- or plate-like shield.

Preferably, the flaps are separated from adjacent sheet material and/or flap(s) along a separation line. The separation lines may be essentially lines along which the sheet material is cut so as to form the one or more flaps. In the present invention, the separation lines are preferably pre-cut, i.e. the flaps formed during the shield before the treatment or procedure, more preferably during the manufacturing process. The pre-formed flaps moot the need for cutting the shield to access target areas. Cutting the shield during use may lead to particle contamination and loss of sterility because of the non-sterile particles released from cutting and/or tearing the device. Nonetheless, it is envisaged within the scope of the invention to provide separation lines which may be easily detached, without contamination, to form the flap prior use, for example by providing means for separating the flap from the sheet or another flap along the separation line(s) prior to use.

In a preferred embodiment, the separation lines are slits. The device may be seen as a sheet comprising a plurality of flaps, or as a sheet comprising a plurality of slits.

Preferably, the device further comprises means for covering the separation lines. The device may comprise covers to protect the separation lines. The covers may be integrally formed with the sheet material, or attached to the shield. A cover may be an elongated strip of material having a shape and dimensions to suitably cover a separation line.

Within the context of the present invention, the barrier to ionising radiation comprises a material or component, which can absorb or block radiation. In a preferred embodiment, the barrier to ionising radiation comprises one or more heavy metals, such as lead. The barrier may comprise a composite material comprising lead, or a lead-free composite material which may include tin, antimony, tungsten, bismuth or other elements. The latter lead-free composite is advantageous in that they are safe for non-hazardous disposal and recycling. More preferably, the barrier comprises one or more lead-free compounds with lead-equivalent properties.

The device according to the present invention may comprise one or more additives, such as one or more antimicrobial layers, antibacterial agents, antifungal agents, antiyeast agents and/or antiviral agents.

The device may comprise means for detecting ionising radiation. This feature is particularly advantageous to monitor radiation exposure in a direct and immediate manner during procedures and treatments.

The device may comprise a compound or component capable of reacting upon exposure to ionizing radiation. The reaction may be to emit light, to emit a radiation at a different wavelength, or to change colour. Such compounds react in a detectable, visible and/or a measurable manner, thereby providing an indication of radiation exposure. This may be useful for monitoring the location and/or intensity of the radiation, and identifying areas e.g. where protection is required, or where radiation is insufficient for imaging or treatment. Preferably, the device comprises a layer of material capable of reacting to radiation exposure. More preferably, the layer is positioned on the surface of the device facing away from the patient, i.e. facing the health professional, in use. This embodiment is particularly useful to detect any default, damage, breach and/or crack in the shield, which would leak radiation. In practice, the radiation is emitted from below the patient, travelled through the patient, and is absorbed or blocked by the shield. In the shield is damaged, radiation would reach the reactive material in the outer layer, thereby signalling a radiation leak to the user.

The device may comprise or be coupled to a radiation sensor, such as a radiation dosimeter.

According to a second aspect of the present invention, there is provided a method for the manufacture of a radiation shield device according to any one of claims 1 to 11, as defined in claim 15

The method may further comprise the step of forming folding lines in the sheet and/or the step of forming covers over the separation lines.

The invention will be further described with reference to the drawings and figures, in which
Figure 1A is a schematic representation of a radiation shield device according to the present invention, in which the flaps are in the shielding configuration;
Figure 1B is a schematic representation of the radiation shield device of figure 1A, in which a flap is in the working configuration;
Figure 2 is a schematic partial representation of exemplary folding lines and folding area;
Figure 3 is a schematic partial representation of an exemplary folding line;
Figures 4A to 4C are schematic partial representations of exemplary spines;
Figures 5, 6 and 7 are schematic representations of radiation shield devices according to the present invention, comprising a peripheral hem;
Figures 8, 9A and 9B are schematic representations of radiation shield devices according to the present invention, comprising separation line covers;
Figures 10A to 10F are schematic representations of exemplary radiation shield devices according to the present invention;
Figure 11 is a schematic representation of a fluoroscopy imaging system;
Figure 12 is a schematic representation of a radiation shield device according to the present invention placed on a patient;

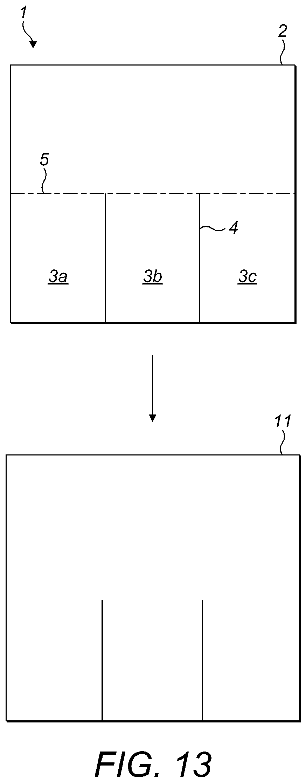

Figure 13 is a schematic representation of a system comprising a radiation shield device and a bag;
Figure 14 is a schematic representation of a further radiation shield device according to the present invention; and
Figures 15A to 15I illustrate examples of in use configurations of a radiation shield according to the present invention.

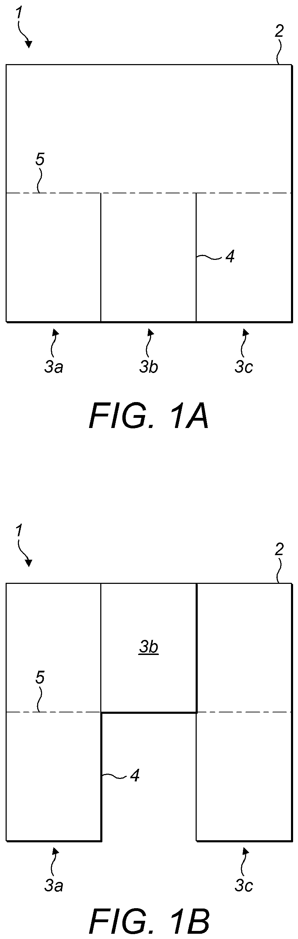

With reference to figure 1A, there is illustrated a radiation shield device 1 comprising a sheet of material 2 comprising a plurality of flaps 3a, 3b, 3c, wherein said sheet 2 comprises a barrier to ionising radiation.

The barrier absorbs and/or blocks ionising radiation, such as x-rays or gamma rays, used in treatment, diagnosis, prognosis and imaging processes. However, the present invention may be used in technological fields, other than the medical, dental and veterinary fields, wherein ionising radiations are used or encountered.

The barrier may comprise one or more radiation blocking and/or absorbing agents. Preferred agents include heavy metals, such as lead, and other compounds and composite materials which are used in radiation PPE. The composite may be a heavy metal-containing composite or may be free of heavy metal, and instead may include one or more of tin, antimony, tungsten, bismuth or other radiation absorbing or blocking elements.

The sheet 2 may be have a monolayer structure, wherein the layer comprises the barrier to ionising radiation. It may have a multilayer structure, wherein, in addition to the one or more radiation barrier layers, it includes other functional or aesthetic layers. The optional layers include structural layers, weighing layers, protective layers, surface layers such as cleanable layers, anti-slip layers, antimicrobial layers, antibacterial layers, antifungal layers, antiyeast layers, antiviral layers, mesh, fabric or textile layers, impermeable layers, and the like. Where the device must be positioned on the patient according to a set direction, the device may comprise coloured layers. For example, where the device comprises a leak detection layer, the colour of the surface facing the patient is preferably different from the colour of the surface facing the health professional. The layers in a multilayer structure may be joined to each other by means of adhesive, co-extrusion, lamination, coating, mechanical attachment (such as sewing), and the like.

The device 1 may be free of lead, so as to be easily disposed of and/or recyclable. The device 1 may be a single use device, and is sterilisable for repeated use.

The shield device 1 is preferably substantially flexible so that it can rest on the body of the patient, substantially following his or her contours. Increasing the rigidity of the sheet (for example to form a plate) could facilitate the folding and unfolding of the flaps 3a, 3b, 3c, however, this would to be to the detriment of manoeuvrability for the health professional, and comfort for the patient.

The device 1 comprises a plurality of flaps 3a, 3b, 3c. Preferably, the sheet 1 and the flaps 3a, 3b, 3c, are made of the same material and/or have the same structure. Although it is envisaged, within the scope of the present invention, that they may be made of different materials and/or have different structures.

In the embodiment illustrated in figures 1A and 1B, the device 1 comprises three flaps 3a, 3b, 3c, however, devices comprising more or fewer flaps are envisaged within the scope of the present invention. The dimensions, shape, location and number of flaps can be set depending on the intended procedure and target area(s).

The flaps 3a, 3b, 3c extend from the sheet 2 and lie, in the shield configuration, in the plane of the sheet 2. The sheet 2 itself may form or may be used as a flap.

A flap may be separated from an adjacent sheet and/or flap along one or more separation lines 4. The device may be provided with pre-detached separations lines 4. Additionally, or alternatively, the device may be provided with separation lines 4 which may allow the user to detach the flaps from adjacent flaps and/or sheets, prior use. The flap may be partially or completely detachable from the device.

A flap may be connected to an adjacent sheet and/or flap by one or more folding lines 5.

A folding line 5 is a line about which the sheet and/or flap can be folded. Figure 1A illustrates a shield device 1 in which the sheet 2 and flaps l are in a shielding configuration. The device 1 lies against the body of the patient, and the sheet 2 and flaps lie substantially in the same place. Figure 1B illustrate the same shield device 1, in which flaps 3a and 3c are in a shielding configuration, and flap 3b has been folded about the folding line 5 into a working configuration to expose a target area on the patient. In the shielding configuration, the flap 3b may lie flat against the sheet 2. The folding line 5 may enable a first flap to be folded upon a sheet or a second flap (i.e. if it is located between the first flap and the sheet or a second flap), or it may enable the first flap to be folded upon itself (i.e. if the folding line 5 is positioned across the flap).

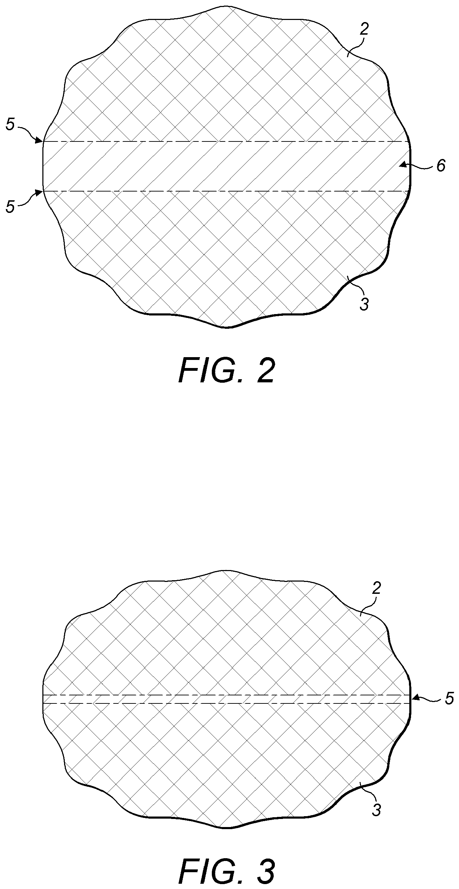

A folding line 5 may form one or more folding areas 6 between two or more folding lines (figure 2), or may be a single line 5 (figure 3).

The folding area 6 may or may not be bordered by folding lines 5. In the latter case, the folding area 6 may be an area of different rigidity than that of the sheet 2 and/or flaps 3a, 3b, 3c. The folding area 6 may for example have a different thickness than the sheet and/or flaps, be made of a different material, and/or comprise one or more layers.

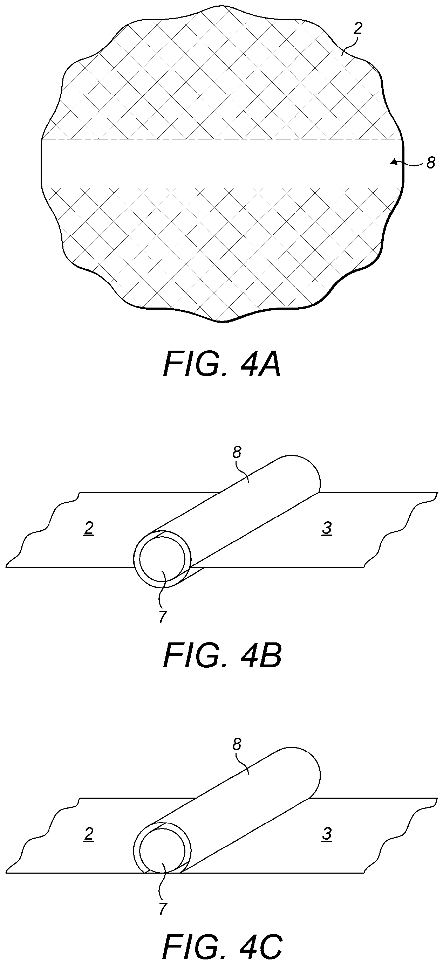

The device 1 may comprise one or more spines or battens 7. As illustrated in figures 4A to 4C, the spine 7 may comprise or consist of an elongated member (band-, baton- or rod-like). A spine or batten 7 may comprise a single elongated member or may comprise a plurality of elongated members (multi-sectional spine or batten). The spine 7 may be secured or adhered to the device 1 by any means known in the art. For example, the spines 7 may be adhered to the device 1 by means of an adhesive or by mechanical means (e.g. sewing, hoops/loops or other fastening means). The spine 7 may be embedded or coupled to the device may any suitable means. In an embodiment, the device 1 may comprise a pocket 8 to accommodate the spine 7, and the pocket 8 may be arranged and configured so that the spine 7 is embedded in the device 1 (figure 4B) or the pocket 8 may be formed substantially on the surface of the device 1 (figure 4C). The spine 7 may comprise or consist of metal, plastics materials, fabric, paper and/or cardboard. The spines or battens 7 facilitate the folding of a flap along a folding line 5, and prevent the folding of a flap onto itself.

The one or more spines or battens 7 may be positioned across a sheet 2 or a flap 3a, 3b, 3c (i.e. transversally; e.g. by extending from on edge of the sheet or flap to an opposing or adjacent edge of the sheet or flap). Additionally or alternatively the one or more spines or battens 7 may be positioned at any one or more edges of the sheet 2 or flap(s) 3a, 3b, 3c.

In an embodiment, the device 1 is provided with a plurality of pockets 8, and removable spines 7, which may be inserted into one or more pockets 8, prior use and depending the intended use or procedure.

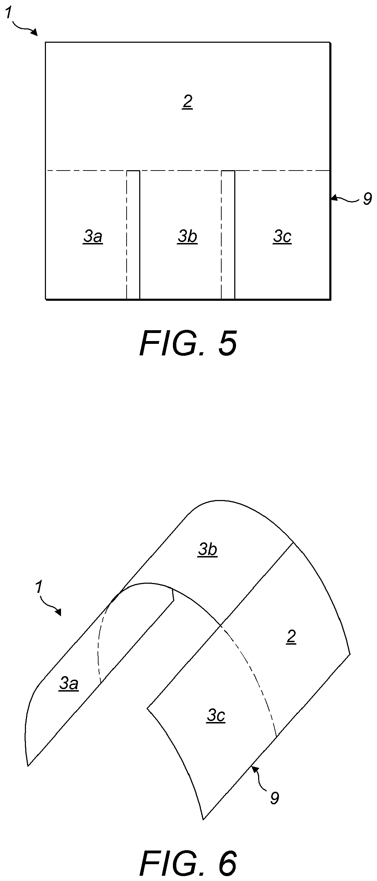

The device 1 comprises a peripheral hem 9 partially or completely along the periphery of the device 1, sheet 2 and/or flaps 3a, 3b, 3c (see figure 5). The hem 9 either has a higher density than the sheet material so as to weigh the device 1 onto the patient, and improve stability, or the peripheral hem 9 comprises a plurality of spines or battens.

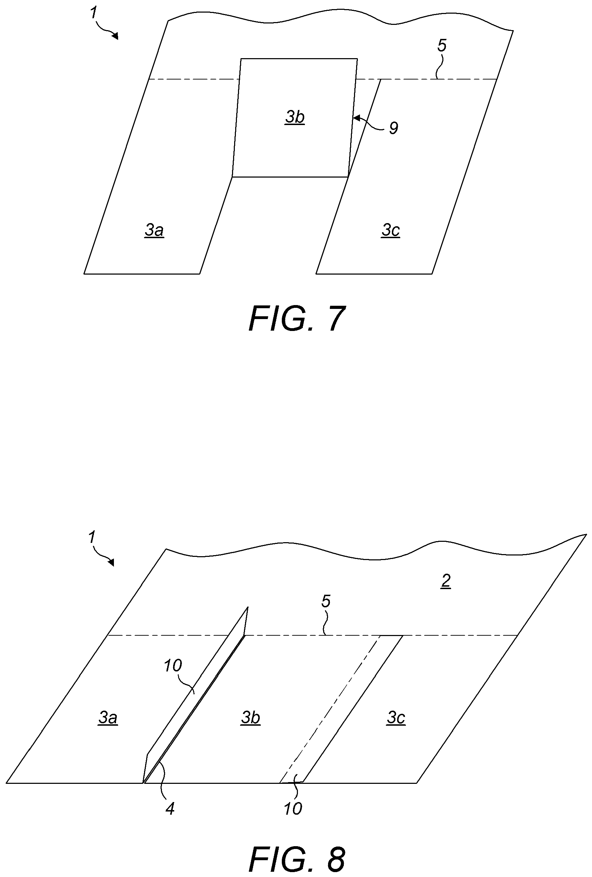

The folding lines 5, folding areas 6, spines 7 and/or hem 9 described herein may be bendable or shapable. This enables the health professional to shape the device 1 to follow the anatomy of the patient, to facilitate access to specific target areas, to increase the protection efficiency of the device 1. Figures 6 and 7 illustrates a device 1 comprising a peripheral hem 9 incorporating a bendable spine 7, which allows the device 1 to be shaped as required.

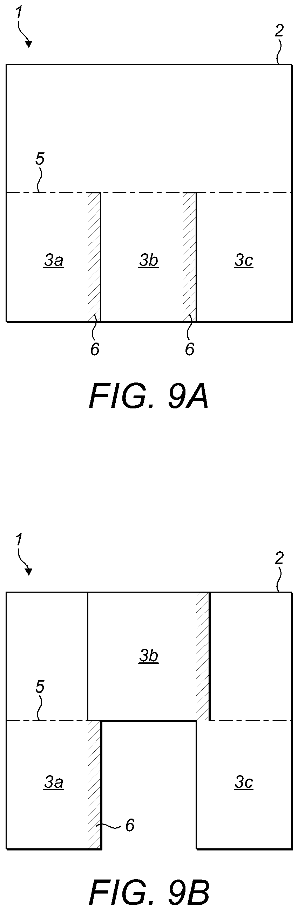

The device 1 comprises one or more separation lines 4 separating adjacent flap(s) and/or sheet(s). A separation line 4 may be protected by a cover 10, as illustrated in figures 8, 9A and 9B.

When placed over the patient, the outer flaps (e.g. 3a and 3c) have a tendency to slide outwardly towards the sides of the patient, thereby leaving gaps at the separation lines 4 between the flaps 3a, 3b, 3c, through which radiation can leak. The spines 7, and more generally the folding lines 5, and/or the hem 9 may act as battens to impart stability to the device 1, and to prevent the flaps 3a, 3c to slide outwardly. Other solutions are proposed to prevent the device 1 from shifting from its intended position, and the device 1 may be provided with means for improving the stability of the device. For example, the device 1 (or bag 11) may be provided with an anti-slip surface, said surface facing, in use, the patient. For example, the device 1 (or bag 11) may be provided with attachment means, to attach the device 1 (or bag 11) to the patient's gown. For example, spines 7 and/or hem 9 may be bendable or shapable so as to be bent or shaped to be secured to the patient. For example, the spines 7 and/or hem 9 may be made of a material which is heavier than the material of the sheet 1 and/or flaps, so as a weigh the device 1 down onto the patient. For example, the device 1, as a whole, may be thick or heavy enough to weigh down onto the patient. For example, the spines or battens 7 may comprise or consist of a magnetic material or element, to prevent two adjacent spines or battens 7 from accidentally pulling apart from each other.

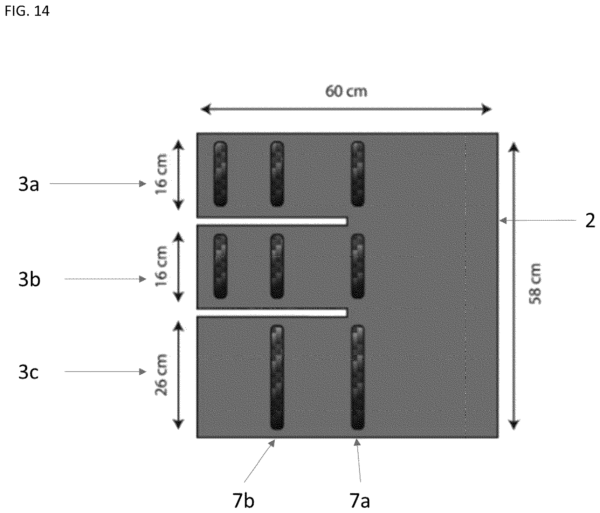

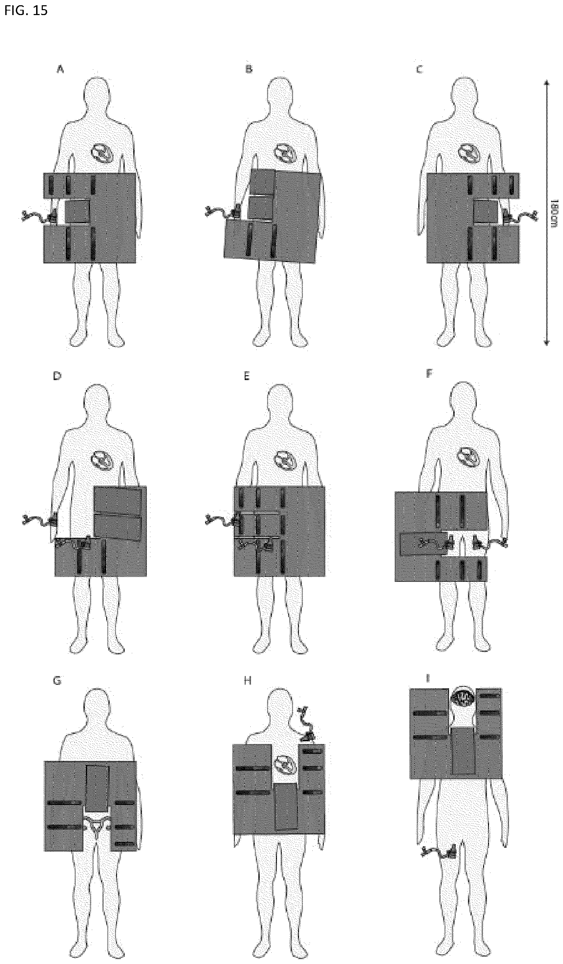

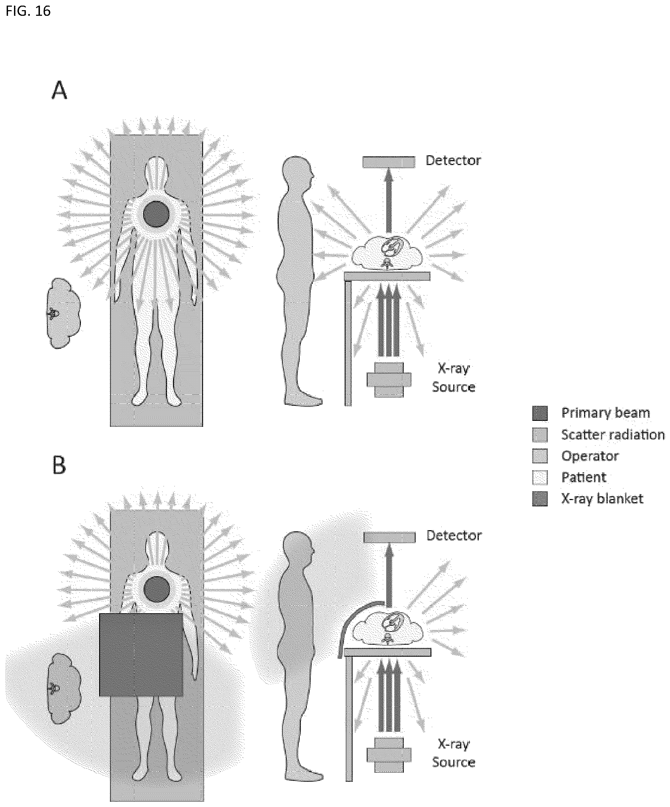

A preferred embodiment is described below with respect to figures 14 and 15A to 15I. The features described with respect to this embodiment may be combined with features of any one of the previously described embodiments.

The device 1 comprises a sheet 2 and three flaps 3a, 3b, 3c. The flaps 3a, 3b, 3c are separated from each other by a separation line 4 (i.e. a slit or cut line).

The device 1 comprises a plurality of battens 7. In this embodiment, each batten 7 comprises or consists an elongated substantially flat member, which preferably comprises a polymeric material. For example, each batten 7 are made of a 2mm wide styrene acrylonitrile polymer. The batten 7 are secured to the device 1, preferably to the surface of the device 1. For example, the battens 7 comprise a layer of adhesive, such as a self-adhesive carbon fiber film. Battens 7a are positioned on or adjacent the folding line between the sheet 2 and a fold 3a, 3b, 3c. In particular, the batten 7a creates a folding line about which a flap 3a, 3b, 3c may be folded about, for example to rest upon the sheet 2. In a similar manner, battens 7b are positioned transversally across the flap(s) 3a, 3b, 3c to create multiple folding lines about which the flaps can be folded, for example upon itself.

The dimensions of the device 1 are preferably 40cm to 120cm long and 40cm to 120cm wide, and more preferably 60cm to 80cm long and 60cm to 80cm wide. Most preferred dimensions are 58cm long and 60cm wide. The flaps may have the same dimensions as each other, or different dimensions.

The present invention is advantageous compared to known sheets used procedures requiring radio-imaging in that each flap can be selectively, and completely or partially, lifted for the medical practitioner to reveal the area to be visualised.

Currently available sheets which do not comprise any opening, need to be fully lifted in order to visualise an area, thereby exposing the medical practitioner to harmful radiations. This problem is emphasized when the practitioner requires visualisation and/or access to several areas in the patient, for example in the case of transcatheter procedures. Such sheets usually have large dimensions in order to shield the practitioner from both direct and reflected radiation, and are therefore cumbersome to manoeuvre to access a small area of the patient. Alternatively, conventional sheets are available of smaller, more easily to manipulate, dimensions, but offer limited protection against radiation to the practitioner.

Other sheets are available with a single "fixed" opening, so that the sheet must be moved on the patient every time a different area of the patient must be accessed. Depending on the position of the sheet on the patient, the sheet will leave large areas of the patient uncovered, thereby exposing the medical practitioner to radiation. Alternatively, the radiation source must be switched off every time the sheet is moved so as to limit exposure to radiation. This is not possible in emergency procedures. Often, these openings do not comprise any sort of cover so that the practitioner is unnecessarily exposed to radiation passing through said opening.

The present invention is advantageous in that only the area to be visualised can be selectively uncovered and all other areas can remain shielded, thereby offering a wide range of possible placements and access/visualisation points.

The flaps 3a, 3b, 3c can be completely or partially lifted depending on the extent of access required. Therefore, the dimensions of the opening can be varied. The battens 7 allows for controlled flexing, and reduces damage to the device 1 and to the integrity of the material. Thus, ruptures and tears are less likely to form so that the device 1 is efficient after repeated uses.

The battens 7 provides some rigidity to the device 1, but more importantly it provides stability, so that the device 1 stays in place on the patient during the procedure. The device 1 need not be secured to the patient or to the operating table.

The flaps 3a, 3b, 3c are preferably made of the same material as the sheet 2. The flaps 3a, 3b, 3c and the sheet 2 are preferably integrally formed. For example, the flaps 3a, 3b, 3c are formed by cutting slits along the separation lines 4. The flaps 3a, 3b, 3c may be sewn or otherwise attached to the sheet 2, provided that they can still perform the function of covering or uncovering an area of the patient (i.e. an opening).

The device 1 may comprise means for detecting, monitoring and/or measuring radiation levels. The sheet 1 may comprise one or more radio-sensitive agents. The agent may be capable of reacting to an emitted radiation, for example, by changing colour upon exposure to a specific radiation and/or at a predetermined level. In an embodiment, the device 1 comprises a layer of material capable of reacting to radiation exposure for example by emitting light or changing colour. The layer is positioned, in use, on the surface of the device facing away from the patient, i.e. facing the health professional. This device 1 is particularly useful to detect any default, damage, breach and/or crack in the shield, which would leak radiation. In practice, the radiation is emitted from below the patient, travelled through the patient, and is absorbed or blocked by the shield. In the shield is damaged, radiation would reach the reactive material in the outer layer, thereby signalling a radiation leak to the user. The reaction level may be quantifiable so that the level of radiation can be determined. The device 1 may comprise a separate component capable of detecting, monitoring and/or measuring radiation levels, for example, a radiation dosimeter.

The pattern of the separating lines 4 and the folding lines 5 can be varied depending on the intended use or procedure and the location and size of the target area. The overall shape and dimensions of the device 1 can be varied depending on the intended use or procedure, on the size and shape of the patient or of the target area.

The flaps 3a,3b,3c provide protection when lying on the patient, and access when the patient is uncovered. The separation lines 4, i.e. the slit between two flaps 3a,3b,3c or between a sheet 2 and a flap3a,3b,3c, themselves may be used to access target areas. The flaps may be separated so as to allow access through the slit, whilst still protecting the health professional from harmful radiation. Thus, the present shield device 1 may be defined as a device 1 comprising a sheet of radiation blocking/absorbing material comprising a plurality of flaps 3a,3b,3c to protect the health professional; and it could also be defined as a device 1 comprising a sheet of radiation blocking/absorbing material comprising a plurality of slits (i.e. the separation lines) to access the target area of the patient.

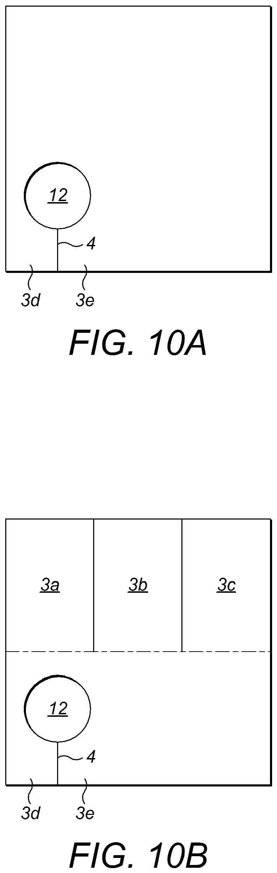

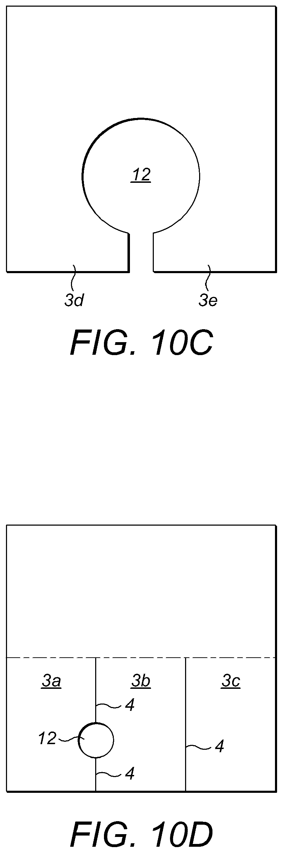

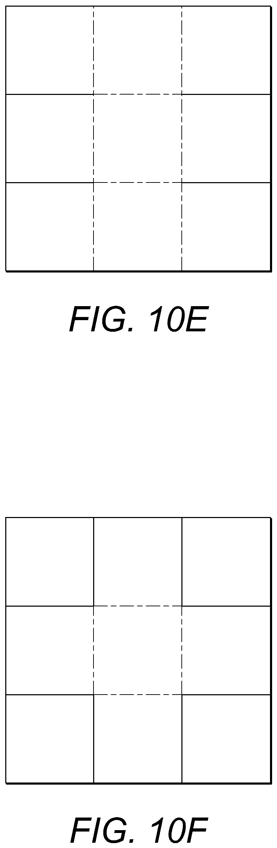

The device 1 may be provided with one or more apertures 12 to allow access to specific target areas, without needing to lift a flap or to pull separation lines apart. As illustrated in figures 10A to 10C, the flaps 3d,3e may define an opening 12 over a target area (figures 10A and 10B, flaps 3d,3c enabling access to the heart, flaps 3a, 3c to access the wrists, flap 3b to access the groin area; figure 10C, for neurosurgery). Figure 10D illustrates a device 1 with an aperture 12 defined at a location along the separation line 4. The device 1 can be rotated or flipped over to allow access to the intended site.

The device 1 preferably has a thickness of 0.1 to 2 mm, when used in medical, dental or veterinary applications. The device 1 may be a single-use or re-usable device. In the case of a re-usable device, the device 1 is preferably washable and is sterilisable. In a preferred embodiment, the device 1 comprises a sterilisable outer layer, or consists of a sterilisable material.

Additionally, or alternatively, there is provided within the context of the present invention, a shielding system comprising a radiation shield device as described herein, and a bag 11 arranged and configured to enclose the shield device. The bag 11 is preferably sterilised or sterilisable. Preferably, the device 1 is removable from the bag 11. The bag 11 may be closable, or its aperture may be folded over (in a pillow-case type construction) so as to preserve the sterility of the device 1. In a preferred embodiment, the bag 11 has substantially the same the outer shape as the device 1 it is intended to enclose. Preferably, the dimensions of the bag 11 are greater than those of the device 1. More preferably, the dimensions of the bag 11 are large enough to enclose the device 1, but small enough to prevent movement of the device 1 within the bag 11. The system allows repeated use of the device 1, as the bag 11 is discarded after each use.

The present invention is particularly useful in procedures during which the health professional must remain in the room when the radiation is emitted. This is the case in surgical and interactional procedures where imaging is needed to guide the surgeon through the patient's anatomy. One such example is fluoroscopy imaging, which enables surgeons to see the patient's internal organs and structures.

Examples of procedures which may require imaging include surgery (e.g. cardiac, vascular, gastrointestinal, pulmonary, thoracic, neurological, orthopaedical, urological, gynaecological), catheter insertion which requires a visualisation of the circulatory system) and device implantation (e.g. ventricular assist devices, stents and pacemakers).

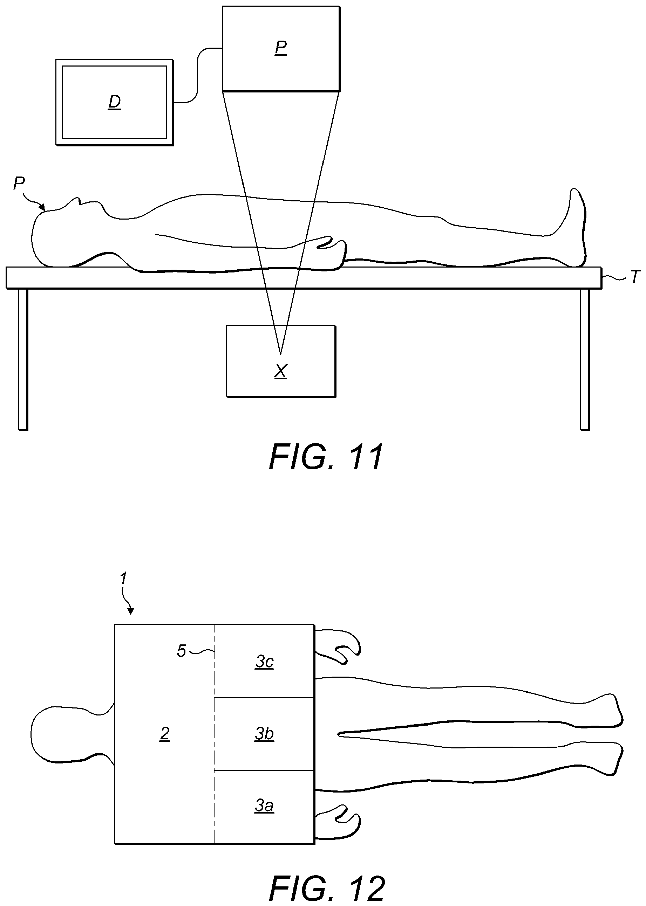

Figure 11 is a basic representation of a fluoroscopy imaging system, comprising a radiation-emitting device X (including for example an X-ray generator, an X-ray tube, a collimator and a filtration device), a table T upon which the patient P lies, an image-processing device P (including for example an image intensifier, an image receptor or camera) and a display monitor D for the surgeon to visualise the captured images.

The advances in non-invasive imaging technology allow for life-saving interventional procedures, with minimal patient discomfort, and vastly replace conventional open surgical procedures. The surgeons are also able to carry out more minute and accurate procedures, in areas which are difficult of access, because partially hidden by the patient's anatomy or because they require passage through small vessels. However, fluoroscopy does involve exposure to harmful radiations, and the present invention seeks to minimise the risks to the surgeons.

In use, the patient is positioned on a table T, and the device 1 is positioned so as to cover the target area(s) of the patient, as illustrated by figure 12. The radiation is emitted by the radiation-emitting device X, travel through the patient P, and are absorbed or blocked by the device 1.

The present invention is particularly useful in procedures requiring access to multiple target areas, the delivery of instruments and/or medical devices to a target area through the patient's anatomy. This is often the case in keyhole and transcatheter procedures, which are favoured over local invasive surgery, such as open-heart surgery. For example, a typical catheter insertion procedure involves several distinct paths and target areas along the patient's circulatory system. Various access sites to the circulatory system are available, including access in or adjacent the groin, the wrist, the neck or subclavian area, the leg. The target site may be any tissue, muscle, organ in the body, including the heart. The present invention may also be used in procedures requiring local access and procedure, such as orthopaedic procedures (e.g. to the hand, or hip).

In a typical transcatheter procedure, the surgeon may start by lifting the flap 3b covering the groin area of the patient to insert the catheter. The flap 3b is folded about the folding line 5, and rested upon sheet 2. The folding line 5 may be a folding line 5 between the flap 3b and the sheet 2 (to enable the flap 3b to be folded upon the sheet 2), or a folding line 5 on the flap 3b (to enable the flap 3b to be folded upon itself). Alternatively, if the flap 3b comprises a bendable peripheral hem 9, the hem 9 may be bent to reveal a smaller area surrounding the target area. The radiation emitted by the radiation-emitting device X can now pass through the patient, and reach the image-processing device P so that the surgeon can visualise the groin area and more particularly the femoral artery and veins. Once the catheter is inserted, its path can be visualised by selectively lifting the flaps and/or sheet. If the access point is found to be unsuitable, then the surgeon may decide to close the flap, and attempt insertion from a different access point on the patient.

Without the device 1, the body areas of the surgeon which are not covered by radiation PPE would be continuously exposed to radiation. By selectively uncovering each target area, the exposure of the surgeon to radiation is minimised. This also means that the health professional requires less or no protective wear.

Once the procedure is completed, the device 1 may be folded along the folding lines 5 and separation lines 4 to be placed into a bag and/or discarded.

### Examples

X-rays are an indispensable tool in many medical procedures and especially for cardiac catheterization. Radiation diagnostics provide the operator with vital information about the patients' anatomy and vascular structures. However, these procedures expose both the patient and the medical staff to potentially harmful ionizing radiation. Although, protection is available, current forms of Personal Protective Equipment (PPE) for medical personnel during the up to thousands of procedures per operator per year are either cumbersome, inadequate or lack functionality leading to inadequate use and possible long-term medical consequences.

The device has been tested and a 93% reduction of radiation-scatter to medical personnel has been observed (compared to using no radiation shield at all).

### Device

1 x 1 metre sheets of 0.5 mm lead material were obtained (CE-marked and conform to IEC 61331-1:2014 standard on protective devices for diagnostic medical X-ray radiation). They were subsequently modified with a sewing machine for prototyping. Battens were made of 2mm styrene acrylonitrile plastic and covered with a self-adhesive carbon fiber film, then fastened with an extra strong 0.8mm waxed polyester thread. The plastic cover was made with commercially available 60x80cm low-density polyethylene bag with a thickness of 50 um. Additional welds to accommodate the flaps were made with a plastic, and the end joints between the flaps were reinforced with spot welds. Sterilization was carried out using vaporized hydrogen peroxide. To validate the sterilization process, chemical indicators were disposed both on the outside and inside of the bags and changed colors to indicate that instruments have been exposed to hydrogen peroxide.

### Exposure

Real life registry data were used on radiation doses from different planes over a year as a template for dosimetry experiments with a human x-ray model. Real time dosimetry was with and without the device were performed during fluoroscopy. By comparing the given radiation with received dose in this setting we found that the device provides protection in a real-life model by 93% over a year. Furthermore, the mat was most effective in settings where operator exposure is highest.

From the above description, it can be seen that the present invention enables optimum protection against radiation, whilst providing the versatility required in medical procedure. As illustrated hereinabove, the shield is versatile and enables health professionals to perform procedures without the concern of exposure. The radiation shield device according to the present invention is a useful addition to the currently available radiation PPE.

Importantly, the device according to the present invention:
- provides optimised protection for medical staff;
- reduces the need for cumbersome PPE wear;
- is easy to integrate into existing medical procedures and set ups; and
- provides multifunctional flaps to selectively access multiple access points and visualisation areas, whilst providing protection against radiation.

Although the present invention has been described within the context of health applications, it is envisaged that it could have other advantageous implementations in fields in which ionising radiations are used and/or encountered.

## Claims

1. Radiation shield device (1) for radiation- and radiation-guided imaging, therapy, diagnosis or prognosis of a subject, wherein said radiation shield device is sterilisable;
wherein said radiation shield device comprises a sheet (2) of material comprising a plurality of flaps (3a, 3b, 3c) configured to be foldable so as to selectively expose a target area of the subject,
wherein said sheet (2) comprises a barrier to ionising radiation;
**characterised in that**
the device comprises a peripheral hem (9) partially or completely along the periphery of the device (1), sheet (2) and/or flaps (3a, 3b, 3c), wherein the hem (9) has a higher density than the sheet material; or a plurality of spines or battens.

2. The device according to claim 1, wherein the device comprises a sterilisable outer layer or consists of a sterilisable material.

3. The device according to claim 1 or 2, wherein the sheet comprises one or more pre-formed lines (5) about which the flaps can be folded.

4. The device according to claim 3, wherein the rigidity of the sheet material is greater than the rigidity of the pre-formed lines.

5. The device according to claim 3, wherein the rigidity of the pre-formed lines is greater than the rigidity of the sheet material.

6. The device according to claim 3, wherein the one or more pre-formed lines comprise a spine (7);
optionally, wherein the spine is made of a bendable material.

7. The device according to any preceding claim, wherein the flaps are separated from adjacent sheet material and/or flap(s) along a separation line;
optionally, wherein the device further comprises means for covering the separation lines.

8. The device according to any preceding claim, wherein the barrier to ionising radiation comprises one or more heavy metals.

9. The device according to any preceding claim, wherein the device comprises one or more antimicrobial layers, antibacterial agents, antifungal agents, anti-yeast agents and/or antiviral agents, or combinations thereof.

10. The device according to any preceding claim, wherein the device further comprises means for detecting ionising radiation.

11. The device according to claim 10, wherein the device comprises a compound or component capable of emitting light upon exposure to ionizing radiation.

12. A system for radiation- and radiation-guided imaging, therapy, diagnosis or prognosis of a subject comprising a radiation shield device (1) according to any preceding claim and a sterilised or sterilisable bag (11) configured to receive the radiation shield device therein.

13. The system according to Claim 12, wherein the bag has substantially the same outer shape as the radiation shield device, and wherein the outer dimensions of the bag are greater than those of the radiation shield device.

14. The system according to Claim 12 or 13, wherein the radiation shield device comprises one or more spines or battens (7).

15. A method for the manufacture of a radiation shield device (1) according to any one of claims 1 to 11, comprising the steps of:
providing a sheet (2) of material comprising a barrier to ionising radiation; and
cutting the sheet along one or more separation lines (4) to form flaps.

16. The method according to claim 15,
further comprising the step of forming folding lines (5) in the sheet; and/or
further comprising the step of forming covers (10) over the separation lines.

## Patentansprüche

1. Strahlungsabschirmungsvorrichtung (1) für die Bestrahlung und die durch Bestrahlung unterstützte Bildgebung, Therapie, Diagnose oder Prognose eines Patienten, wobei die Strahlungsabschirmungsvorrichtung sterilisierbar ist;
wobei die Strahlungsabschirmungsvorrichtung eine Materialbahn (2) mit mehreren Klappen (3a, 3b, 3c) umfasst, die konfiguriert sind, um gefaltet zu werden, um einen Zielbereich des Patienten selektiv zu belichten,
wobei die Bahn (2) eine Barriere gegen ionisierende Strahlung umfasst;
**dadurch gekennzeichnet, dass** die Vorrichtung einen peripheren Saum (9) teilweise oder vollständig entlang des Umfangs der Vorrichtung (1), eine Bahn (2) und/oder Klappen (3a, 3b, 3c) umfasst, wobei der Saum (9) eine höhere Dichte als die Materialbahn aufweist; oder eine Vielzahl von Graten oder Latten.

2. Vorrichtung nach Anspruch 1, wobei die Vorrichtung eine sterilisierbare äußere Schicht umfasst oder aus einem sterilisierbaren Material besteht.

3. Vorrichtung nach Anspruch 1 oder 2, wobei die Bahn eine oder mehrere vorgeformte Linien (5) umfasst, um die die Klappen gefaltet werden können.

4. Vorrichtung nach Anspruch 3, wobei die Steifigkeit der Materialbahn größer ist als die Steifigkeit der vorgeformten Linien.

5. Vorrichtung nach Anspruch 3, wobei die Steifigkeit der vorgeformten Linien größer ist als die Steifigkeit der Materialbahn.

6. Vorrichtung nach Anspruch 3, wobei die eine oder mehreren vorgeformten Linien einen Grat (7) umfassen;
optional, wobei der Grat aus einem biegsamen Material hergestellt ist.

7. Vorrichtung nach einem vorstehenden Anspruch, wobei die Klappen von angrenzendem Materialbahnen und/oder Klappe(n) entlang einer Trennlinie getrennt werden;
optional, wobei die Vorrichtung weiter Mittel zum Abdecken der Trennlinien umfasst.

8. Vorrichtung nach einem vorstehenden Anspruch, wobei die Barriere gegen ionisierende Strahlung ein oder mehrere Schwermetalle umfasst.

9. Vorrichtung nach einem vorstehenden Anspruch, wobei die Vorrichtung eine oder mehrere antimikrobielle Schichten, antibakterielle Mittel, Antimykotika, Antipilzmittel und/oder antivirale Mittel oder Kombinationen davon umfasst.

10. Vorrichtung nach einem vorstehenden Anspruch, wobei die Vorrichtung weiter Mittel zum Nachweis ionisierender Strahlung umfasst.

11. Vorrichtung nach Anspruch 10, wobei die Vorrichtung eine Verbindung oder Komponente umfasst, die bei Bestrahlung mit ionisierender Strahlung Licht emittieren kann.

12. System für die Bestrahlung und die durch Bestrahlung unterstützte Bildgebung, Therapie, Diagnose oder Prognose eines Patienten, umfassend eine Strahlungsabschirmungsvorrichtung (1) nach einem der vorstehenden Ansprüche und einen sterilisierten oder sterilisierbaren Beutel (11), der konfiguriert ist, um die Strahlungsabschirmungsvorrichtung darin aufzunehmen.

13. System nach Anspruch 12, wobei der Beutel im Wesentlichen die gleiche äußere Form wie die Strahlungsabschirmungsvorrichtung hat, wobei die äußeren Abmessungen des Beutels größer sind als die der Strahlungsabschirmungsvorrichtung.

14. System nach Anspruch 12 oder 13, wobei die Strahlungsabschirmungsvorrichtung einen oder mehrere Grate oder Latten (7) umfasst.

15. Verfahren zur Herstellung einer Strahlungsabschirmungsvorrichtung (1) nach einem der Ansprüche 1 bis 11, umfassend die Schritte:
Bereitstellen einer Bahn (2) aus einem Material, das eine Barriere gegen ionisierende Strahlung bildet; und
Schneiden der Bahn entlang einer oder mehrerer Trennlinien (4), um Klappen zu bilden.

16. Verfahren nach Anspruch 15,
weiter umfassend den Schritt des Ausbildens von Faltlinien (5) in der Bahn; und/oder
weiter umfassend den Schritt des Ausbildens von Abdeckungen (10) über den Trennlinien.

## Revendications

1. Dispositif (1) de protection contre les rayonnements pour l'imagerie, la thérapie, le diagnostic ou le pronostic, par rayonnements et guidés par rayonnements, d'un sujet, dans lequel ledit dispositif de protection contre les rayonnements est stérilisable ;
dans lequel ledit dispositif de protection contre les rayonnements comprend une feuille (2) de matériau comprenant une pluralité de rabats (3a, 3b, 3c) configurés pour être pliables de façon à exposer sélectivement une zone cible du sujet,
dans lequel ladite feuille (2) comprend une barrière aux rayonnements ionisants ;
**caractérisé en ce que** le dispositif comprend un bord périphérique (9) partiellement ou complètement le long de la périphérie du dispositif (1), de la feuille (2) et/ou des rabats (3a, 3b, 3c), dans lequel le bord (9) présente une masse volumique supérieure à celle du matériau de la feuille ; ou une pluralité d'épines ou de lattes.

2. Dispositif selon la revendication 1, dans lequel le dispositif comprend une couche externe stérilisable ou consiste en un matériau stérilisable.

3. Dispositif selon la revendication 1 ou la revendication 2, dans lequel la feuille comprend une ou plusieurs lignes préformées (5) autour desquelles les rabats peuvent être pliés.

4. Dispositif selon la revendication 3, dans lequel la rigidité du matériau de la feuille est plus grande que la rigidité des lignes préformées.

5. Dispositif selon la revendication 3, dans lequel la rigidité des lignes préformées est plus grande que la rigidité du matériau de la feuille.

6. Dispositif selon la revendication 3, dans lequel les une ou plusieurs lignes préformées comprennent une épine (7) ;
facultativement, dans lequel l'épine est constituée d'un matériau pliable.

7. Dispositif selon une quelconque revendication précédente, dans lequel les rabats sont séparés du matériau de feuille adjacent et/ou du (des) rabat(s) le long d'une ligne de séparation ;
facultativement, dans lequel le dispositif comprend en outre un moyen pour couvrir les lignes de séparation.

8. Dispositif selon une quelconque revendication précédente, dans lequel la barrière aux rayonnements ionisants comprend un ou plusieurs métaux lourds.

9. Dispositif selon une quelconque revendication précédente, dans lequel le dispositif comprend un(e) ou plusieurs couches antimicrobiennes, agents antimicrobiens, agents antifongiques, agents anti-levure et/ou agents antiviraux, ou des combinaisons de ceux-ci.

10. Dispositif selon une quelconque revendication précédente, dans lequel le dispositif comprend en outre un moyen pour détecter des rayonnements ionisants.

11. Dispositif selon la revendication 10, dans lequel le dispositif comprend un composé ou un composant capable d'émettre de la lumière lors de l'exposition à des rayonnements ionisants.

12. Système pour l'imagerie, la thérapie, le diagnostic ou le pronostic, par rayonnements et guidés par rayonnements, d'un sujet comprenant un dispositif (1) de protection contre les rayonnements selon une quelconque revendication précédente et une poche (11) stérilisée ou stérilisable configurée pour recevoir le dispositif de protection contre les rayonnements dans celle-ci.

13. Système selon la revendication 12, dans lequel la poche présente sensiblement la même forme externe que le dispositif de protection contre les rayonnements, et dans lequel les dimensions externes de la poche sont plus grandes que celles du dispositif de protection contre les rayonnements.

14. Système selon la revendication 12 ou la revendication 13, dans lequel le dispositif de protection contre les rayonnements comprend une ou plusieurs épines ou lattes (7).

15. Procédé de fabrication d'un dispositif (1) de protection contre les rayonnements selon l'une quelconque des revendications 1 à 11, comprenant les étapes de :
fourniture d'une feuille (2) de matériau comprenant une barrière aux rayonnements ionisants ; et
découpe de la feuille le long d'une ou plusieurs lignes de séparation (4) pour former des rabats.

16. Procédé selon la revendication 15,
comprenant en outre l'étape de formation de lignes de pliage (5) dans la feuille ; et/ou
comprenant en outre l'étape de formation d'éléments de couverture (10) sur les lignes de séparation.
